# EUROPEAN PATENT APPLICATION

(11) **EP 4 837 976 A1**
(43) Date of publication of application: **11.11.2026**
(21) Application number: 24898269.6
(22) Date of filing: 22.05.2024
(51) Int. Cl.: C12Q 1/6888

(54) **PORK QUALITY EVALUATION KIT AND PORK QUALITY DETERMINATION METHOD USING SAME**

(30) Priority: 27.11.2023 KR 20230166426
(71) Applicant: Republic of Korea (Management: Rural Development Administration), Deokjin-gu Jeonju-si, Jeollabuk-do 54875 (KR)
(72) Inventor: CHO, In Cheol, Wanju-gun Jeonbuk-do 55365 (KR); KIM, Hyeon Ah, Wanju-gun Jeonbuk-do 55365 (KR); KANG, Yong Jun, Wanju-gun Jeonbuk-do 55365 (KR); KIM, Sang Geum, Wanju-gun Jeonbuk-do 55365 (KR); YOO, Ji Hyun, Wanju-gun Jeonbuk-do 55365 (KR); KIM, Yoo Kyung, Wanju-gun Jeonbuk-do 55365 (KR); KIM, Su Yeon, Wanju-gun Jeonbuk-do 55365 (KR)
(74) Representative: Mewburn Ellis LLP
(86) International application number: PCT/KR2024/095834
(87) International publication number: WO 2025/116677

(57) **Abstract**

The present invention relates to a diagnostic kit for meat quality of pigs comprising a composition for diagnosing meat quality of pigs, the composition comprising a first primer set and a second primer set, wherein the composition for diagnosing meat quality of pigs is in powder form.

## Description

### TECHNICAL FIELD

### Cross-Reference to Related Application

This application claims the benefit of priority to Korean Patent Application No. 2023-0166426, filed on November 27, 2023, the entire contents of which are incorporated herein by reference.

The present invention relates to a diagnostic kit for meat quality of pigs and a method for determining meat quality of pigs using the same.

### BACKGROUND ART

The pig industry has mainly focused on improving productivity in terms of the quantity of meat and on the distribution of fresh meat, which has been accompanied by a decrease in pork quality (Cameron, 1990; Cliplef and McKay, 1993). In Korea, grilled pork and boiled pork account for most pork consumption, and thus fresh meat is consumed in large quantities.

In general, meat quality is an important selection criterion in purchasing meat. Nonuniform meat quality decreases consumer confidence and reduces consumers' willingness to select high-quality meat (Jung et al., 2011). Accordingly, in order to overcome this problem, attempts have been made to develop a meat quality trait improvement system so as to satisfy consumer demand for pork quality (Lee et al., 2012).

Recently, in order to improve the quality of pork, pigs have been raised under certain standards and scientifically managed, and the pork obtained therefrom has been branded, with various brands of pork already being commercially sold. However, the general public has difficulty visually distinguishing such branded pork from non-branded pork.

Meanwhile, most economic traits in pigs are known to be quantitative traits expressed under the influence of various genes, and studies on identifying causative genes involved in such traits and investigating genetic loci have been actively conducted. In particular, for identification of trait-related genetic markers, studies on identification of single nucleotide polymorphisms (SNPs) in functional genes and associations thereof with traits have been actively conducted, and various trait-related SNPs have been identified to date.

However, an analysis method based on PCR (Polymerase Chain Reaction)-RFLP (Restriction Fragment Length Polymorphism) for predicting meat quality traits of pork using SNP markers requires a process of treating PCR-amplified products with restriction enzymes and diagnosing genotypes. Therefore, approximately two days are required until final analysis, and thus the method could not be applied to field analysis, and analysis costs are increased due to the use of restriction enzymes. In addition, since the possibility of errors in genotype interpretation is high depending on the activity of the restriction enzyme used or the treatment time, there is also a problem in that additional analysis needs to be performed.

Accordingly, there remains a need in the art for a method capable of accurately determining the meat quality of pork based on a PCR technique without using restriction enzymes.

### [PRIOR ART DOCUMENTS]

### [PATENT DOCUMENTS]

(Patent Document 1) Korean Patent No. 10-1941893 (published on January 25, 2019)
(Patent Document 2) Korean Patent No. 10-2019997 (published on September 10, 2019)

### DISCLOSURE OF THE INVENTION

### TECHNICAL PROBLEM

An object of the present invention is to provide a diagnostic kit for meat quality of pigs including a composition for diagnosing meat quality of pigs, which is capable of rapidly and accurately determining meat quality of pigs using a difference in length of an amplification product derived from a causative gene for meat quality of pigs.

Another object of the present invention is to provide a method for rapidly and accurately determining meat quality of pigs using a kit including a primer set that includes primers prepared using the MYH3 gene, which is a causative gene for meat quality of pigs.

### TECHNICAL SOLUTION

One aspect of the present invention provides a diagnostic kit for meat quality of pigs including a composition for diagnosing meat quality of pigs, the composition including a first primer set and a second primer set, wherein the composition for diagnosing meat quality of pigs is in powder form.

In the present invention, the first primer set may consist of nucleotide sequences represented by SEQ ID NOs: 1 and 2, and the second primer set may consist of nucleotide sequences represented by SEQ ID NOs: 3 and 4.

In the present invention, the composition may generate an amplification product exhibiting a difference in length upon electrophoresis by amplifying the target gene MYH3.

In the present invention, when the length of an amplification product is 311 bp, the pork may be determined to be of the low-meat-quality type; when the lengths of amplification products are 311 bp and 650 bp, the pork may be determined to be of the intermediate-meat-quality type; and when the length of an amplification product is 650 bp, the pork may be determined to be of the high-meat-quality type.

In the present invention, meat quality may be related to one or more meat quality trait factors selected from intramuscular fat content, meat color, shear force, water-holding capacity, and fatty acid composition.

In the present invention, the composition for diagnosing meat quality of pigs may further include a reaction buffer, Taq DNA polymerase (Taq polymerase), dNTPs, and loading dye.

In the present invention, the kit may further include instructions for use.

Another aspect of the present invention provides a method for determining meat quality of pigs, including: a) extracting genomic DNA from a pig sample; b) performing an amplification reaction using the isolated genomic DNA as a template and a diagnostic kit for meat quality of pigs; and c) performing electrophoresis on the amplification products, identifying a difference in size between the amplification products, and determining the meat quality of pork.

In the present invention, as a result of electrophoresis in step c), when the amplification product appears as a single band of 311 bp, the pork may be determined to be of the low-meat-quality type; when the amplification product appears as a single band of 650 bp, the pork may be determined to be of the high-meat-quality type; and when both bands of 311 bp and 650 bp appear, the pork may be determined to be of the intermediate-meat-quality type.

### ADVANTAGEOUS EFFECTS

According to the present invention, the meat quality of pigs can be accurately determined by grade as the low-meat-quality type, the intermediate-meat-quality type, and the high-meat-quality type merely by determining a difference in length of amplification products by electrophoresis after PCR amplification without restriction enzyme treatment.

The present invention has the advantages of accurately determining the meat quality of pigs within a short period of approximately 3 hours, as a determination result can be obtained by PCR amplification followed by electrophoresis, and of significantly reducing analysis costs because expensive restriction enzymes are not used.

In addition, the present invention can determine the meat quality of pigs without errors in interpretation of the genotype of a causative gene for meat quality through PCR amplification alone, and thus is convenient because additional or auxiliary analysis does not need to be separately performed.

In addition, the present invention can prevent errors occurring in the previous simplified meat quality diagnostic method while accurately determining the meat quality of pigs by grade as the low-meat-quality type, the intermediate-meat-quality type, and the high-meat-quality type.

In addition, unlike conventional liquid samples, the composition of the diagnostic kit of the present invention is provided in powder form, thereby facilitating distribution and storage.

### BRIEF DESCRIPTION OF THE DRAWINGS

FIG. 1 is a diagram illustrating a diagnostic kit for meat quality of pigs according to the present invention.
FIG. 2 is a diagram illustrating a method for diagnosing meat quality of pigs using a diagnostic kit for meat quality of pigs according to the present invention.
FIG. 3 is a diagram illustrating variations within a gene locus for predicting meat quality of pigs through next-generation sequencing (NGS) analysis.
FIG. 4 is a diagram illustrating candidate genes associated with meat quality of pigs within the locus identified in the Ensembl database.
FIG. 5 is a diagram illustrating a variant region within the gene locus.
FIG. 6 is a diagram illustrating results of determining meat quality of pigs using the kit according to the present invention.
FIG. 7 is a diagram illustrating results of comparing methods for determining meat quality of pigs using the conventional HpyCH4-RFLP method, the conventional simplified determination composition, and the kit according to the present invention.

### BEST MODE FOR CARRYING OUT THE INVENTION

Hereinafter, embodiments of the present invention will be described in detail with reference to the accompanying drawings so that those skilled in the art to which the present invention pertains can readily practice the present invention. However, the present invention may be implemented in various different embodiments and is not limited to the embodiments described herein. Like reference numerals are used to designate like parts throughout the specification.

As used herein, when terms such as "about," "approximately," or "at least" are used in relation to numerical values in the present specification, such terms are intended to allow for a theoretical, experimental, statistical, or empirical error of ±10%, ±7%, ±5%, ±3%, ±2%, or ±1% based on the corresponding numerical value.

As a result of intensive research efforts to develop a method for determining meat quality of pigs based on genetic traits, the present inventors have identified the MYH3 gene as a gene that determines meat quality traits of pigs, and have confirmed that genetically determined meat quality traits of pigs can be predicted using the same.

In the present invention, the term "MYH3 gene" refers to a gene encoding myosin heavy chain 3, which is one of the heavy chain proteins included in myosin constituting the muscles of animals, and the nucleotide sequence thereof can be obtained from publicly known databases such as the GenBank database of NCBI (GenBank Accession Nos. KX549311 (LAND) and KX549312 (KNP)).

The present inventors have confirmed that the MYH3 gene determines meat quality traits of intramuscular fat content and redness in pigs, and have found that genetic variation exists between native breeds having excellent meat quality traits and foreign breeds not having excellent meat quality traits, and thus that the meat quality traits of pigs can be determined by detecting a gene marker containing the variation.

In this regard, as a result of performing QTL analysis and Linkage and linkage disequilibrium (LALD) mapping for meat quality traits of pigs on offspring obtained by crossing Jeju native pigs with Landrace or Duroc pigs, the MYH3 gene present on chromosome 12 was found to be a causative gene involved in meat quality traits of pigs.

Meanwhile, the "PCR-RFLP" method is one of various DNA analysis techniques for improving genetic characteristics or performance of livestock, which analyzes restriction fragment length polymorphism of gene fragments treated with a restriction enzyme in combination with PCR technology, and is a method that, when a restriction enzyme recognition site is present at a specific point mutation site, enables the genotype of an analysis target to be determined more rapidly and accurately by utilizing the fact that the lengths of fragments generated by cleavage with the restriction enzyme vary according to the genotype difference of each individual.

The PCR-RFLP method is also widely used for analyzing meat quality of pigs. However, according to this method, approximately 2 days are required from gene amplification to genotype determination, which limits its applicability to on-site analysis. In addition, since such a conventional analysis method uses a restriction enzyme, there is always a possibility of error in determination depending on the activity or other conditions of the restriction enzyme, and there is also a problem in that analysis cost increases due to the use of the restriction enzyme.

Accordingly, based on the MYH3 gene identified as a causative gene for meat quality, the present inventors designed primers capable of accurately diagnosing meat quality of pigs by grade, namely the low-meat-quality type, the intermediate-meat-quality type, and the high-meat-quality type, based only on analysis of differences in the lengths of amplification products by PCR amplification and electrophoresis, and confirmed that the primer sets thus designed can readily and rapidly determine meat quality of pigs.

In addition, the present inventors discovered that some errors occurred in previous simplified diagnosis method and, as a result of intensive research efforts thereon, designed primer sets using a new variant region rather than the existing variant region, and found that the errors occurring in the previous simplified diagnosis method could be resolved, thereby completing the present invention.

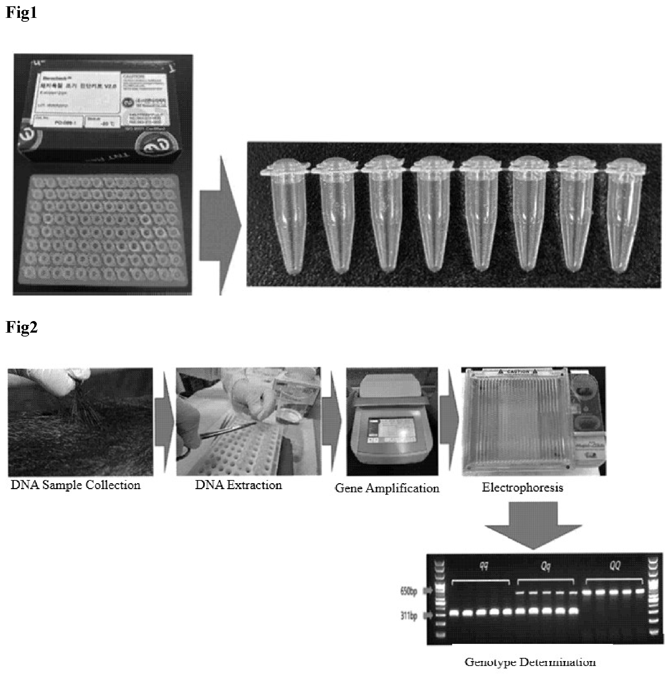

FIG. 1 is a diagram illustrating a diagnostic kit for meat quality of pigs according to the present invention.

Referring to FIG. 1, an aspect of the present invention provides a diagnostic kit for meat quality of pigs including a composition for diagnosing meat quality of pigs including a first primer set and a second primer set, wherein the composition for diagnosing meat quality of pigs is in powder form.

In the present invention, the first primer set may consist of nucleotide sequences represented by SEQ ID NOs: 1 and 2, and the second primer set may consist of nucleotide sequences represented by SEQ ID NOs: 3 and 4.

In addition, in the present invention, since the composition for diagnosing meat quality of pigs is in powder form, the composition is easier to distribute and store than conventional liquid samples, and diagnosis can be performed without problems even when left at room temperature for 5 days or more, preferably 7 days or more.

As used herein, the term "primer" refers to a short nucleotide sequence having a free 3' hydroxyl group, which can form base pairs with a complementary template and functions as a starting point for copying a template strand. In the present invention, the primer used for amplification of a genetic marker may be a single-stranded oligonucleotide capable of acting as a starting point for template-directed DNA synthesis under appropriate conditions, such as an appropriate buffer and other appropriate components (e.g., four different nucleoside triphosphates) and a polymerizing agent such as a DNA or RNA polymerase or a reverse transcriptase, at a suitable temperature; the appropriate length of the primer may vary depending on the intended use. The primer sequence need not be completely complementary to a polynucleotide including the genetic marker or a polynucleotide complementary thereto, and can be used as long as the primer is sufficiently complementary to hybridize thereto.

In addition, the primer may be modified. Specific examples thereof may include methylation, capping, substitution of nucleotides, or modification between nucleotides, for example, modification with an uncharged linkage (e.g., methyl phosphonate, phosphotriester, phosphoramidate, carbamate, or the like) or a charged linkage (e.g., phosphorothioate, phosphorodithioate, or the like).

In the present invention, each primer may be a polynucleotide consisting of a nucleotide sequence represented by SEQ ID NOs: 1, 2, 3, or 4.

Those skilled in the art can design an appropriate variant based on the sequence information (including the order of linkage and sequence length of nucleotides) of the primers disclosed in the present invention, which consist of the nucleotide sequences represented by SEQ ID NOs: 1, 2, 3, or 4. Therefore, the scope of the primers according to the present invention is not necessarily limited to primers having the nucleotide sequences of SEQ ID NOs: 1, 2, 3, or 4.

The primers constituting the primer sets according to the present invention can amplify the target gene, the MYH3 gene, to generate an amplification product exhibiting a difference in length upon electrophoresis.

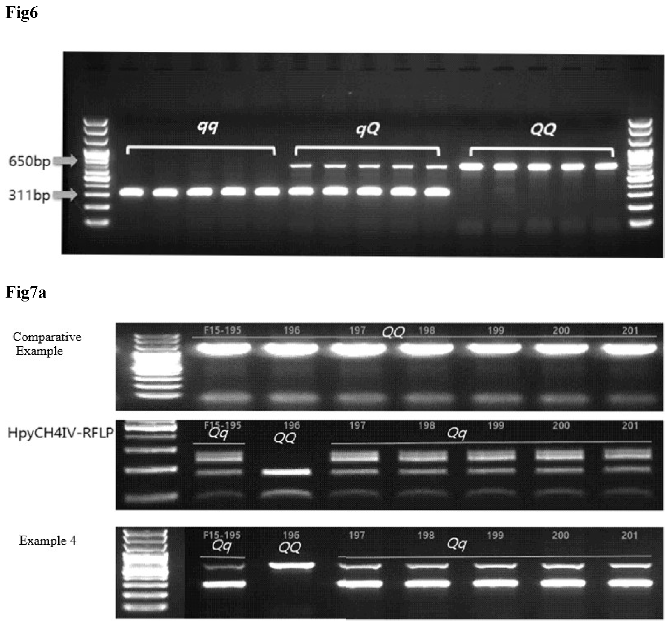

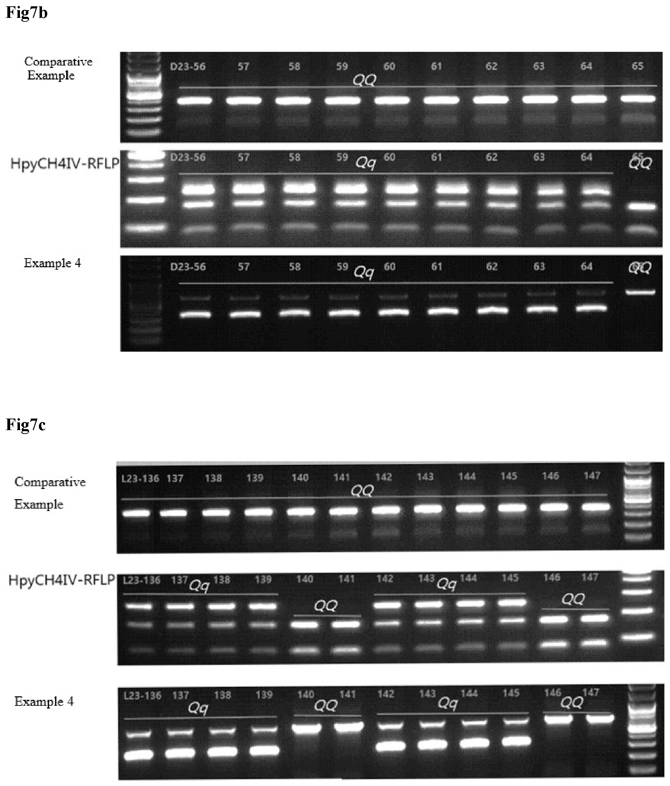

In one embodiment of the present invention, it was confirmed that, after amplifying the target gene related to meat quality of pigs or meat quality traits, i.e., the MYH3 gene, using a kit including a composition including a first primer set consisting of the nucleotide sequences represented by SEQ ID NOs: 1 and 2 and a second primer set consisting of the nucleotide sequences represented by SEQ ID NOs: 3 and 4, and then performing electrophoresis, meat quality of pigs can be determined according to differences in the lengths of the resulting amplification products (FIGS. 6 and 7).

Specifically, as a result of amplifying the target gene, i.e., the MYH3 gene, using the kit according to the present invention and then performing electrophoresis, when the amplification product has a length of 311 bp, the pork can be determined to be of the low-meat-quality type (*qq*); when the amplification products have lengths of 311 bp and 650 bp, the pork can be determined to be of the intermediate-meat-quality type (*Qq*); and when the amplification product has a length of 650 bp, the pork can be determined to be of the high-meat-quality type (*QQ*).

Accordingly, the diagnostic kit for meat quality of pigs according to the present invention can accurately diagnose, determine, or predict the meat quality grade of a pig to be analyzed in a more subdivided manner, i.e., as the high-meat-quality type, the intermediate-meat-quality type, and the low-meat-quality type, using only PCR amplification followed by electrophoresis, and can shorten the analysis time and enable application to on-site analysis. In addition, since the present invention does not use a restriction enzyme, the analysis cost can also be reduced.

According to an embodiment of the present invention, the aforementioned meat quality of pigs may be related to factors associated with meat quality traits, including, but not limited to, intramuscular fat content, meat color, shear force, water-holding capacity, and fatty acid composition.

As used herein, the term "meat quality trait" refers to various phenotypic traits representing the condition of carcasses, excluding bones, obtained from pigs, and the phenotypic traits may include, but are not limited to, intramuscular fat content, meat color, water-holding capacity, and shear force. Intramuscular fat content refers to the content of fat contained in muscle; meat color refers to the color of meat as visually determined; water-holding capacity refers to the ability of meat to retain water; and shear force refers to the degree of toughness of meat when torn. Meat quality may be determined to be higher as the intramuscular fat content is higher, the meat color is redder, and the water-holding capacity and shear force are lower.

In the present invention, the composition for diagnosing meat quality of pigs may further include Taq DNA polymerase (Taq polymerase), dNTPs, and loading dye, and Taq DNA polymerase (Taq polymerase), dNTPs, and loading dye may be used without limitation as long as they are widely known in the art.

In the present invention, the composition for diagnosing meat quality of pigs, Taq DNA polymerase (Taq polymerase), dNTPs, and loading dye may be mixed in the following amounts.

Specifically, 0.1 to 0.5 µL of Taq DNA polymerase (Taq polymerase), 1 to 3 µL of dNTPs, 0.1 to 1 µL each of the forward and reverse primers of the two primer sets included in the composition for diagnosing meat quality of pigs, for a total of 0.4 to 4 µL, 1 to 3 µL of loading dye, and 10 to 15 µL of DW (distilled water) may be added to a tube, reacted, and then prepared in powder form using a freeze dryer.

Preferably, 0.3 µL of Taq DNA polymerase (Taq polymerase), 2 µL of dNTPs, 0.75 µL each of the forward and reverse primers of the two primer sets included in the composition for diagnosing meat quality of pigs, 2 µL of loading dye, and 12.7 µL of DW (distilled water), for a total of 20 µL, may be added to a tube, reacted, and then prepared in powder form using a freeze dryer, and the meat quality of pigs may be readily determined within the above ranges.

In the present invention, the kit may include instructions for use. The kit according to the present invention can determine the level of meat quality traits of pigs by identifying the MYH3 marker gene, which is a causative gene for meat quality of pigs, as well as other marker genes, through PCR amplification, or by identifying mRNA expression levels of the marker genes.

In addition, in the present invention, the kit may further include a test tube or other suitable container.

In addition, in the present invention, the kit can determine the meat quality of pigs by adding sterile distilled water and a DNA sample to the kit, followed by PCR and electrophoresis.

FIG. 2 is a diagram illustrating a method for diagnosing meat quality of pigs using a diagnostic kit for meat quality of pigs according to the present invention.

In another aspect, the present invention provides a method for determining meat quality of pork, including: a) extracting genomic DNA from a pig sample; b) performing an amplification reaction using the separated genomic DNA as a template and a diagnostic kit for meat quality of pigs; and c) performing electrophoresis on the amplification products, identifying a difference in size between the amplification products, and determining the meat quality of pork.

In step b), when the diagnostic kit for meat quality of pigs is used, the amplification reaction may be performed by adding genomic DNA and distilled water.

In the method for determining meat quality of pigs according to the present invention, the pig sample may be a sample such as hair, urine, blood, various body fluids, isolated tissue, isolated cells, or saliva, but the pig sample is not particularly limited to these examples.

As used herein, the term "nucleic acid" refers to not only DNA, but also cDNA synthesized from mRNA, and RNA molecules.

In one embodiment of the present invention, the primer set included in the kit used in step b) may be prepared based on the MYH3 gene.

In one embodiment of the present invention, the electrophoresis in step c) may be performed on a commonly used agarose gel. The electrophoresis may be performed within 50 minutes, preferably within 40 minutes, more preferably within 30 minutes.

In one embodiment of the present invention, as a result of the electrophoresis in step c), when the amplification product appears as a single band of 311 bp, the pork may be determined to be of the low-meat-quality type; when the amplification product appears as a single band of 650 bp, the pork may be determined to be of the high-meat-quality type; and when both bands of 311 bp and 650 bp appear, the pork may be determined to be of the intermediate-meat-quality type.

In one embodiment of the present invention, the entire process of determining meat quality of pigs according to the present invention may vary depending on the condition of the sample, the skill level of the equipment operator, and the like, but the determination of meat quality of pigs may be completed within approximately 3 hours, and at the latest within 5 hours.

### MODE FOR CARRYING OUT THE INVENTION

Hereinafter, the present invention will be described in more detail through examples. However, these examples are provided for illustrative purposes only, and the scope of the present invention is not limited to these examples.

### Example 1. Preparation of Pigs

The experimental animals used in the present invention were 32 pigs of the low-meat-quality type (11), 46 pigs of the intermediate-meat-quality type (12), and 30 pigs of the high-meat-quality type (22), for a total of 108 pigs, which were prepared as shown in Table 1 and Table 2 below.

**[Table 1]**

| 11 | | 12 | | 22 | |
|---|---|---|---|---|---|
| 1 | LD21-1 | 25 | F15-85 | 49 | F15-75 |
| 2 | LD21-3 | 26 | F15-96 | 50 | F15-80 |
| 3 | LD21-7 | 27 | F15-103 | 51 | F15-89 |
| 4 | LD21-17 | 28 | F15-108 | 52 | F15-91 |
| 5 | D23-1 | 29 | LD21-4 | 53 | F15-97 |
| 6 | D23-18 | 30 | LD21-8 | 54 | F15-101 |
| 7 | D23-23 | 31 | LD21-14 | 55 | F15-109 |
| 8 | D23-45 | 32 | LD21-18 | 56 | F15-114 |
| 9 | L22-60 | 33 | L22-84 | 57 | LD21-2 |
| 10 | L22-72 | 34 | L22-89 | 58 | LD21-23 |
| 11 | L22-80 | 35 | L22-93 | 59 | LD21-31 |
| 12 | L22-87 | 36 | L23-48 | 60 | LD21-37 |
| 13 | L23-73 | 37 | L23-52 | 61 | L23-110 |
| 14 | L23-78 | 38 | L23-74 | 62 | L23-116 |
| 15 | L23-84 | 39 | L23-82 | 63 | L23-122 |
| 16 | L23-128 | 40 | L23-85 | 64 | D22-40 |
| 17 | D22-36 | 41 | D22-35 | 65 | D22-56 |
| 18 | D22-43 | 42 | D22-51 | 66 | D23-6 |
| 19 | D22-46 | 43 | D22-63 | 67 | D23-17 |
| 20 | D22-50 | 44 | D23-4 | 68 | D23-24 |
| 21 | D22-59 | 45 | D23-5 | 69 | D23-39 |
| 22 | D22-65 | 46 | D23-16 | 70 | D23-53 |
| 23 | D22-68 | 47 | D23-43 | 71 | D23-55 |
| 24 | D22-71 | 48 | D23-46 | 72 | D23-65 |

**[Table 2]**

| 11 | | 12 | | 22 | |
|---|---|---|---|---|---|
| 1 | D20-195 | 9 | D20-84 | 31 | DK21-288 |
| 2 | D20-200 | 10 | D20-90 | 32 | DK21-289 |
| 3 | D22-65 | 11 | D20-106 | 33 | DK21-290 |
| 4 | D22-67 | 12 | D20-107 | 34 | DK21-291 |
| 5 | DK21-332 | 13 | D20-108 | 35 | DK21-293 |
| 6 | DK21-333 | 14 | D20-112 | 36 | DK21-296 |
| 7 | DK21-334 | 15 | D20-114 | | |
| 8 | DK21-335 | 16 | D22-64 | | |
| | | 17 | D22-69 | | |
| | | 18 | DK21-81 | | |
| | | 19 | DK21-92 | | |
| | | 20 | DK21-292 | | |
| | | 21 | DK21-294 | | |
| | | 22 | DK21-295 | | |
| | | 23 | DK21-336 | | |
| | | 24 | DK21-337 | | |
| | | 25 | DK21-338 | | |
| | | 26 | DK21-339 | | |
| | | 27 | DK21-340 | | |
| | | 28 | DK21-341 | | |
| | | 29 | DK21-342 | | |
| | | 30 | DK21-343 | | |

### Example 2. DNA Isolation

DNA was extracted from the jugular vein of the pigs (test animals) used in this study or from the tails of piglets produced therefrom and was used for genetic analysis. DNA isolation was performed using a modified sucrose-proteinase K method (Sambrook et al., 1989). The absorbance of the isolated DNA was measured using a NanoDrop ND-1000 spectrophotometer (NanoDrop Technologies, USA), and genomic DNA having an A260/A280 ratio of 1.8 or higher was used as a template for polymerase chain reaction (PCR).

### Example 3. Extraction and Analysis of Nucleotide Sequences Including Primers for Determining Meat Quality of Pigs

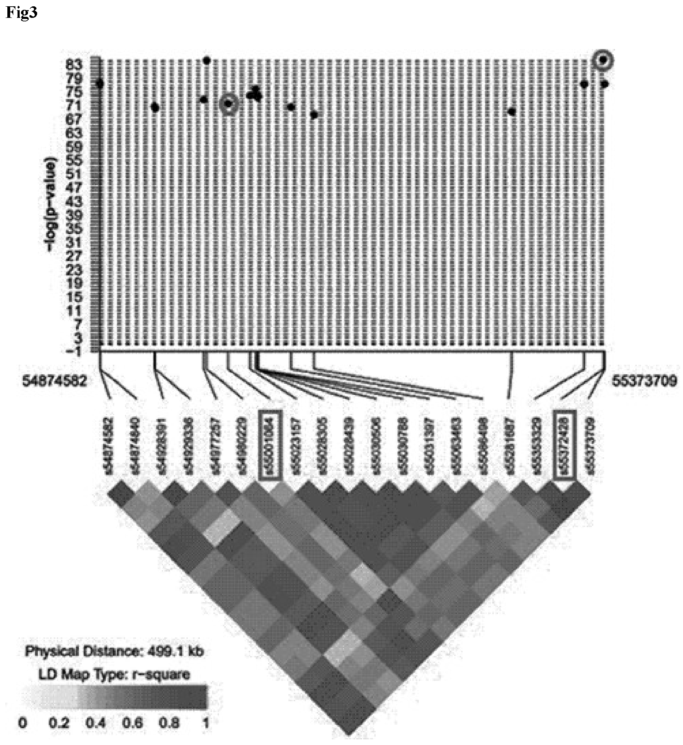

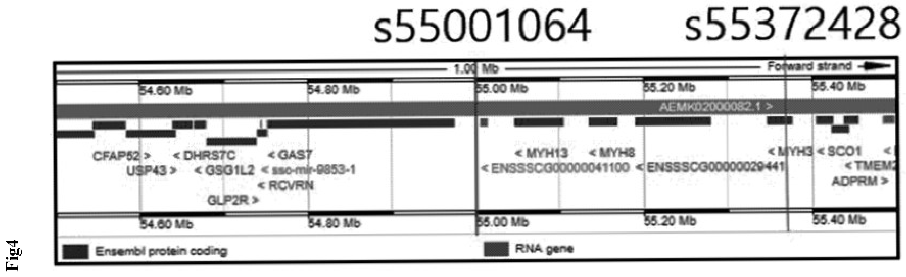

Through next-generation sequencing (NGS) analysis, a region (s55372428) having a higher P-value than the existing SNP for determining the high-meat-quality type (s55373709) was discovered (FIGS. 3 and 4; Table 3). As a result of gene function analysis, it was confirmed that the MYH3 gene is the causative gene that determines meat quality (intramuscular fat content and red meat) of pigs, and through functional analysis, the SNP at position 55373709 was confirmed to be the causative variant, and the distance between the two SNPs was confirmed to be about 1.3 kb.

**[Table 3]**

| **SNP NAME** | **pvalue** | **PP** |
|---|---|---|
| s54928391 | 2.50E-71 | 0.2033 |
| s54929336 | 6.00E-71 | 0.2179 |
| s54980229 | 6.55E-85 | 0.0536 |
| s55001064 | 3.31E-72 | 0.7553 |
| s55023157 | 1.32E-74 | 0.3297 |
| s55028305 | 1.56E-76 | 0.1188 |
| s55028439 | 1.13E-74 | 0.0602 |
| s55030506 | 4.03E-74 | 0.2072 |
| s55030788 | 3.13E-74 | 0.2091 |
| s55063463 | 3.05E-71 | 0.1978 |
| s55086498 | 4.55E-69 | 0.0814 |
| s55281687 | 6.08E-70 | 0.2432 |
| s55372428 | 3.08E-85 | 0.6782 |
| s55373709 | 4.52E-78 | 0.0247 |

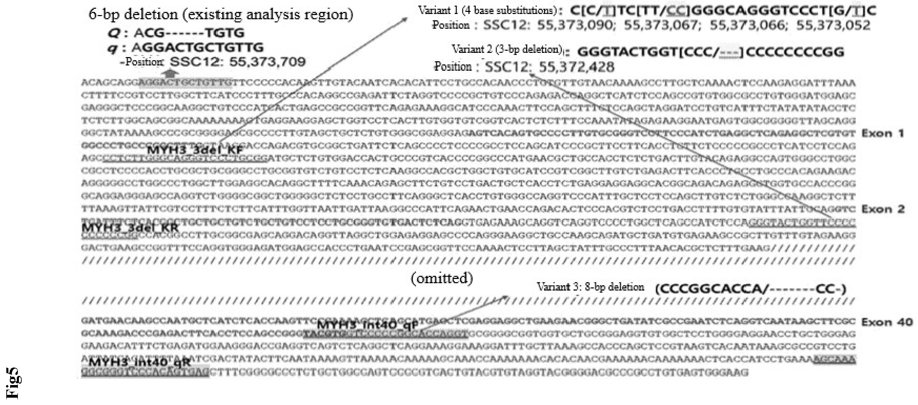

**Example 4. Preparation of Primers for Determining Meat Quality of Pigs and Preparation of a Kit for Determining Meat Quality of Pigs** Based on the sequence information (Table 4) of the MYH3 gene including the SNPs discovered in Example 3 (variant 1 (SSC12: 55,373,090; 55,373,067; 55,373,066; 55,373,052), variant 2 (SSC12: 55,372,428), and variant 3), primers were prepared as follows (FIG. 5). Then, a first primer set consisting of SEQ ID NOs: 1 and 2, a second primer set consisting of SEQ ID NOs: 3 and 4, Taq DNA polymerase (Genet Bio, Prime Taq DNA Polymerase), dNTPs (Genet Bio, Prime Taq DNA Polymerase), and loading dye (Genet Bio, Prime Taq DNA Polymerase) were mixed. Specifically, 0.3 µL of Taq DNA polymerase, 2 µL of dNTPs, 0.75 µL each of the forward and reverse primers of the first primer set consisting of SEQ ID NOs: 1 and 2 and the second primer set consisting of SEQ ID NOs: 3 and 4, for a total of 3 µL, 2 µL of loading dye, and 12.7 µL of DW (distilled water), for a total of 20 µL, were added to a tube, reacted, and then prepared in powder form using a freeze dryer. Freeze-drying was performed at -20°C, and the composition was prepared in powder form in a 50 µL PCR tube, thereby preparing the kit.

**[Table 4]**

| |
|---|
| SEQ ID NO: 9: MYH3 (ENSSSCG00000018007) |
| ACAGCAGGAGGACTGCTGTTGTTCCCCCACAAGTTGTACAATCACACATTCCTGCCACAACCCTGTGTTGTAACAAAAG CCTTGCTCAAAACTCCAAGAGGATTTAAACTTTTCCGTCCTTGGCTTCATCCCTTTGCCCACAGGCCGAGATTCTAGGT CCCCGCTGTCCCAGAGACCAGGCTCATCTCCAGCCGTGTGGCGCCTGTGGATGGAGCGAGGGCTCCCGGCAAGGCTGT CCCATCACTGAGCCGCCGGTTCAGAGAAAGGCATCCCAAACTTCCAGCTTTCTCCAGCTAGGATCCTGTCATTTCTATA TATACCTCTCTCTTGGCAGCGGCAAAAAAAAGTGAGGAAGGAGCTGGTCCTCACTTGTGGTGTCGGTCACTCTCTTTCC AAATATAGAGAAGGAATGAGTGGCGGGGGTTAGCAGGGGCTATAAAAGCCCGCGGGGAGCGCCCCTTGTAGCTGCTCTG TGGGCGGAGGAGAGTCACAGTGCCCCTTGTGCGGGTCCTTCCCATCTGAGGCTCAGAGGCTCGTGTGGCCCTGCCCGGC TTTGGTAAGGACCAGACGTGCGGCTGATTCTCAGCCCCTCCCCGCCTCCAGCATCCCGCTTCCTTCACCTGTTCTCCCC CGCCCTCATCCTCCAGAGCCCTCTTGGGCAGGGTCCCTGCGGATGCTCTGTGGACCACTGCCCGTCACCCCGGCCCATG AACGCTGCCACCTCTCTGACTTGTACAGAGGCCAGTGGGCCTGGCCGCCTCCCCACCTGCGCTGCGGGCCTGCGGTGTC TGTCCTCTCAAGGCCACGCTGGCTGTGCATCCGTCGGCTTGTCTGAGACTTCACCCTGCCTGCCCACAGAAGACAGGGG GCCTGGCCCTGGCTTGGAGGCACAGGCTTTTCAAACAGAGCTTCTGTCCTGACTGCTCACCTCTGAGGAGGAGGCACGG CAGACAGAGGGTGGTGCCACCCGGGCAGGAGGGAGCCAGGTCTGGGGCGGCTGGGGGCTCTCCTGCCTTCAGGGCTCAC CTGTGGGCCAGGTCCCATTTGCTCCTCCAGCTTGTCTCTGGGCCAAGGCTCTTTTAAAGTTATTCGTCCTTTCTCTTCA TTTGGTTAATTGATTAAGGCCCATTCAGAACTGAACCAGACACTCCCACGTCTCCTGACCTTTTGTGTATTTATTGCAG GTCTGATTTCTCACGGCTGCTGCTGTCTGCTGTCCTCCTGCGGGTGTGACTCTCAGGTGAGAAAGCAGGTCAGGTCCCC TGGCTCAGCCATCTCCAGGGTACTGGTTCCCCCCCCCCGGCCACGGCCTTGCGGCGAGCAGGACAGGTTAGGCTGGAGA GGAGCCCCAGGGAAGGCTGCCAAGCAGATGCTGATGTGAGAAGCCGCTTGTTTGTAGAAGGGACTGAAGCCGGTTTCCA GGTGGGAGATGGAGCCACCCTGAATCCGAGCGGTTCCAAAACTCCTTAGCTATTTGCCCTTTAACACGCTCTTTGAAG/ |
| /////////////////////////////////////////////////////////////////////////////// //////////////////////////////////////// |
| (omitted) |
| /////////////////////////////////////////////////////////////////////////////// ///////////////////////////// |
| GATGAACAAGCCAATGCTCATCTCACCAAGTTCCGAAAAGCTCAGCATGAGCTCGAGGAGGCTGAAGAACGGGCTGATA TCGCCGAATCTCAGGTCAATAAGCTTCGCGCAAAGACCCGAGACTTCACCTCCAGCCGGGTACGTGGTCCCCCGGCACC AGGTGCGGGGCGGTGGTGCTGCGGGAGGTGTGGCTCCTGGGGAGGAACCTGCTGGGAGGAAGACATTTCTGAGATGGAA GGGACCGAGGTCAGTCTCAGGCTCAGGAAAGGAAAGGATTTGCTTAAAGCCACCCAGCTCCGTAAGTCACAATAAAGCG CCGTCCTGATTATCACATTTTAAATCGACTATACTTCAATAAAAGTTAAAAACAAAAAGCAAACCAAAAAAACACACAA CGAAAAAACAAAAAAACTCACCATCCTGAAAAGCAAAGGCGGGTCCCACAGTGAGCTTTCGGCGCCCTCTGCTGGCCAG TCCCCGTCACTGTACGTGTAGGTACGGGGACGCCCGCCTGTGAGTGGGAAG |

Primer design:
MYH3_3del_KF: CTT-CCC-GGG-CAG-GGT-CCC-TTC (SEQ ID NO: 1)
MYH3_3del_KR: CCG-GGG-GGG-AAC-CAG-TAC-CC (SEQ ID NO: 2)
MYH3_int40_qF: TAC-GTG-GTC-CCC-CGG-CAC-CAG-GT (SEQ ID NO: 3)
MYH3_int40_qR: CTC-ACT-GTG-GGA-CCC-GCC-TTT-GCT (SEQ ID NO: 4)

### Comparative Example. Preparation of a Composition for Determining Meat Quality of Pigs

Based on the sequence information (Table 4) of the MYH3 gene including the SNP discovered in Example 3 (existing analysis region (SSC12: 55,393,709)), primers were prepared as follows (FIG. 7). Then, a third primer set consisting of SEQ ID NOs: 5 and 6, a fourth primer set consisting of SEQ ID NOs: 7 and 8, Taq DNA polymerase (Genet Bio, Prime Taq DNA Polymerase), dNTPs (Genet Bio, Prime Taq DNA Polymerase), and loading dye (Genet Bio, Prime Taq DNA Polymerase) were mixed. Specifically, 0.3 µL of Taq DNA polymerase, 2 µL of dNTPs, 0.75 µL each of the forward and reverse primers of the third primer set consisting of SEQ ID NOs: 5 and 6 and the fourth primer set consisting of SEQ ID NOs: 7 and 8, for a total of 3 µL, 2 µL of loading dye, and 12.7 µL of DW (distilled water), for a total of 20 µL, were added to a tube, reacted, and then prepared in powder form using a freeze dryer. Freeze-drying was performed at -20°C, and the composition was prepared in powder form in a 50 µL PCR tube, thereby preparing the kit.

Primer design:
MYH3_1Q_F: AGG-ACG-TGT-GTT-CCC-CAC-AC (SEQ ID NO: 5)
MYH3_1Q_R: GGA-ACC-AGT-ACC-CTG-GAG-ATG-G (SEQ ID NO: 6)
MYH3_1q_F: ACg-GCT-GAC-ACC-AGA-ATG-AAC (SEQ ID NO: 7)
MYH3_1q_R: GGG-GAA-CAA-CAG-CAG-TCC-TCC (SEQ ID NO: 8)

### Test Example 1. Determination of Meat Quality Using the Kit According to the Present Invention

Using 15 pork samples (5 pigs of the low-meat-quality type, 5 pigs of the intermediate-meat-quality type, and 5 pigs of the high-meat-quality type), PCR amplification and analysis were performed using the kit prepared in Example 4.

Specifically, 2.0 µL of DNA isolated from pig blood and 18 µL of sterile deionized water were added to the kit, and PCR reactions were performed as shown in Table 5 below. PCR amplification was performed using a mastercycler X50 (Eppendorf, Germany). The amplification products were subjected to electrophoresis on a 0.8 to 1.2% agarose gel, and the genotype was identified under UV light based on whether gene amplification had occurred and the length of the amplification product.

**[Table 5]**

| Temp(°C) | Time | Cycles |
|---|---|---|
| 95 | 5 min | 1 |
| 95 | 10 sec | 30 |
| 65 | 15 sec | |
| 72 | 30 sec | |
| 72 | 5 min | 1 |
| 25 | forever | 1 |

As shown in FIG. 6, the 311 bp band appeared specifically in the 5 pigs of the low-meat-quality type (*qq*), the 650 bp band appeared specifically in the 5 pigs of the high-meat-quality type (*QQ*), and both the 311 bp and 650 bp bands appeared in the 5 pigs of the intermediate-meat-quality type (*Qq*).

### Test Example 2. Analysis of the Accuracy of the Method for Determining Meat Quality of Pigs According to the Present Invention

Using 107 pork samples (31 pigs of the low-meat-quality type, 46 pigs of the intermediate-meat-quality type, and 30 pigs of the high-meat-quality type), PCR amplification and analysis were performed using the kits according to Example 4 and the Comparative Example, and the genotypes were determined using the conventional PCR-RFLP method.

Specifically, 2.0 µL of DNA isolated from pig blood and 18 µL of sterile deionized water were added to the kit, and PCR reactions were performed as shown in Table 5 above. PCR amplification was performed using a mastercycler X50 (Eppendorf, Germany). The amplification products were subjected to electrophoresis on a 0.8 to 1.2% agarose gel, and the genotype was identified under UV light based on whether gene amplification had occurred and the length of the amplification product.

The PCR-RFLP method was performed as follows. Specifically, the portion containing the nucleotide variation was amplified using primers (forward: 5'-TGG TCT TTC CTA ATT GGT GAC AT-3' (SEQ ID NO: 10) and reverse: 5'-AGT TTT GAG CAA GGC TTT TGT T-3' (SEQ ID NO: 11)). PCR reactions were performed in a volume of 20 µL, as shown in Table 5 above, by adding 2.0 µL of DNA isolated from the blood of each pig, 10.0 µL of 2X HS Prime Taq Premix, 0.5 µL each of the forward and reverse primers, 2.0 µL of DMSO (dimethyl sulfoxide), and 5 µL of sterile deionized water. PCR amplification was performed using a mastercycler X50 (Eppendorf, Germany).

The amplified PCR products were digested using the restriction enzyme HpyCH4IV (recognition site: A▼CGT). The restriction enzyme reaction was performed, according to the supplier's instructions, by mixing 3 µL of the PCR amplification product, 1 µL of 10X buffer, 0.3 µL of the restriction enzyme, and 5.7 µL of distilled water, and reacting the mixture at 37°C for 12 hours. Electrophoresis was performed on a 1.5% agarose gel containing EtBr (ethidium bromide) to confirm the cleavage pattern of the MYH3 gene by the restriction enzyme. The results are shown in Table 6 below and FIG. 7.

**[Table 6]**

| Classification | No. of Samples Analyzed | *myh3_genotype* | | |
|---|---|---|---|---|
| | | *qq* | *Qq* | *QQ* |
| Comparative Example | 107 | 11 | 40 | 56 |
| HpyCH4-RFLP | 107 | 31 | 46 | 30 |
| Example 4 | 107 | 31 | 46 | 30 |

| | | | | |
|---|---|---|---|---|
| *qq:* low-meat-quality type, *Qq:* intermediate-meat-quality type, *QQ:* high-meat-quality type | | | | |

Referring to FIG. 7 and Table 6, it was confirmed that errors occurred in all of the low-meat-quality type, the intermediate-meat-quality type, and the high-meat-quality type when the kit according to the Comparative Example was used, whereas the kit according to the present invention produced the same results as the conventional HpyCH4IV-RFLP method.

In addition, as a result of the analysis of differences in amplification product length based on PCR amplification and electrophoresis according to the present invention, it was confirmed that pork of the low-meat-quality type (*qq*) exhibited a 311 bp band, pork of the intermediate-meat-quality type (*Qq*) exhibited both the 311 bp and 650 bp bands, and pork of the high-meat-quality type (*QQ*) exhibited a 650 bp band.

### INDUSTRIAL APPLICABILITY

The present invention is capable of accurately determining, within a short time, the meat quality of pork by grade as the low-meat-quality type, the intermediate-meat-quality type, and the high-meat-quality type, merely by determining differences in the lengths of amplification products by electrophoresis after PCR amplification without restriction enzyme treatment, and can exhibit superior effects as compared with existing primer sets.

## Claims

1. A diagnostic kit for meat quality of pigs comprising a composition for diagnosing meat quality of pigs, the composition comprising a first primer set and a second primer set, wherein the composition for diagnosing meat quality of pigs is in powder form.

2. The diagnostic kit for meat quality of pigs according to claim 1, wherein the first primer set consists of nucleotide sequences represented by SEQ ID NOs: 1 and 2, and the second primer set consists of nucleotide sequences represented by SEQ ID NOs: 3 and 4.

3. The diagnostic kit for meat quality of pigs according to claim 1, wherein the composition is capable of amplifying a target gene, i.e., the MYH3 gene, to generate an amplification product exhibiting a difference in length upon electrophoresis.

4. The diagnostic kit for meat quality of pigs according to claim 3, wherein the composition determines the pork to be of the low-meat-quality type when the amplification product has a length of 311 bp, determines the pork to be of the intermediate-meat-quality type when the amplification products have lengths of 311 bp and 650 bp, and determines the pork to be of the high-meat-quality type when the amplification product has a length of 650 bp.

5. The diagnostic kit for meat quality of pigs according to claim 1, wherein the meat quality is related to one or more meat quality trait factors selected from intramuscular fat content, meat color, shear force, water-holding capacity, and fatty acid composition.

6. The diagnostic kit for meat quality of pigs according to claim 1, wherein the composition for diagnosing meat quality of pigs further comprises Taq DNA polymerase (Taq polymerase), dNTPs, and loading dye.

7. The diagnostic kit for meat quality of pigs according to claim 1, wherein the kit further comprises instructions for use.

8. A method for determining meat quality of pigs, comprising: a) extracting genomic DNA from a pig sample; b) performing an amplification reaction using the separated genomic DNA as a template and the kit according to claim 1; and c) performing electrophoresis on the amplification products and determining meat quality of pork by identifying a difference in size between the amplification products.

9. The method for determining meat quality of pigs according to claim 8, wherein, as a result of the electrophoresis in step c), the pork is determined to be of the low-meat-quality type when the amplification product appears as a single band of 311 bp, is determined to be of the high-meat-quality type when the amplification product appears as a single band of 650 bp, and is determined to be of the intermediate-meat-quality type when both bands of 311 bp and 650 bp appear.
